# EUROPÄISCHE PATENTSCHRIFT

(11) **EP 1 220 662 B1**
(45) Veröffentlichungstag und Bekanntmachung des Hinweises auf die Patenterteilung: **06.07.2005**
(21) Anmeldenummer: 00966123.2
(22) Anmeldetag: 06.10.2000
(51) Int. Cl.: A61K 9/70, A61K 47/12, A61K 31/135

(54) **TRANSDERMALES THERAPEUTISCHES SYSTEM MIT EINEM GEHALT AN TULOBUTEROL-HYDROCHLORID ZUR VERABREICHUNG DES BRONCHODILATATORS TULOBUTEROL ÜBER DIE HAUT**
TRANSDERMAL THERAPEUTIC SYSTEM CONTAINING TULOBUTEROL HYDROCHLORIDE FOR ADMINISTERING THE BRONCHODILATOR TULOBUTEROL VIA THE SKIN
SYSTEME THERAPEUTIQUE TRANSDERMIQUE A BASE D'HYDROCHLORURE DE TULOBUTEROL UTILISE POUR ADMINISTRER LE BRONCHODILATATEUR TULOBUTEROL PAR L'INTERMEDIAIRE DE LA PEAU

(30) Priorität: 16.10.1999 DE 19950066
(43) Veröffentlichungstag der Anmeldung: 10.07.2002
(73) Patentinhaber: LTS Lohmann Therapie-Systeme AG, 56626 Andernach (DE)
(72) Erfinder: BRACHT, Stefan, 56299 Ochtendung (DE)
(74) Vertreter: Flaccus, Rolf-Dieter, Dr.
(86) Internationale Anmeldenummer: PCT/EP2000/009788
(87) Internationale Veröffentlichungsnummer: WO 2001/028531

(56) Entgegenhaltungen:
- EP-A- 0 439 180
- EP-A- 0 922 453

## Beschreibung

Die Erfindung betrifft ein transdermales therapeutisches System mit einem Gehalt an Tulobuterol-hydrochlorid, welches die Verabreichung des Bronchodilatators Tulobuterol über die Haut ermöglicht. Die transdermale Arzneizubereitung ist zur Behandlung von Asthma-Erkrankungen geeignet.

Tulobuterol ist ein Arzneistoff aus der Gruppe der β-Sympathomimetika. Es wirkt überwiegend auf die β₂-Rezeptoren der glatten Muskulatur, beispielsweise in den Bronchien. Aufgrund seiner Eigenschaft, den Bronchialmuskeltonus herabzusetzen und eine Erschlaffung der Bronchialmuskulatur herbeizuführen, wird Tulobuterol zur Therapie asthmatischer Erkrankungen eingesetzt.

Neben oralen Darreichungsformen sind aus der Literatur auch transdermale Applikationssysteme für Tulobuterol bekannt, die erfolgreich bei der Asthma-Therapie eingesetzt werden können. Transdermale therapeutische Systeme, welche Tulobuterol enthalten, sind beispielsweise in JP 63-10716 A, US 5 254 348, US 5 312 627, US 5 571 530 und US 5 639 472 beschrieben worden.

Die US 5 254 348 offenbart ein transdermales therapeutisches System, dessen tulobuterolhaltige Matrix auf der Basis eines Styrol-1,3-dien-styrol-Blockcopolymers oder eines Styrol-Isopren-Styrol-Blockcopolymers aufgebaut ist. Die US 5 312 627 beschreibt ein transdermales therapeutisches System, welches für die Verabreichung von Wirkstoffen mit bronchodilatatorischer Wirkung, z. B. Tulobuterol, geeignet ist. Als Matrix-Polymer wird Polyisobutylen verwendet.

In der US 5 571 530 wird ebenfalls eine perkutane Zusammensetzung mit dem Wirkstoff Tulobuterol beschrieben, wobei der Wirkstoff sich in einer Polymermatrix befindet, die aus einer Mischung von Polyisobutylenen besteht.

Aus der US 5 639 472 ist eine Tulobuterol-enthaltende Zusammensetzung bekannt, welche für die perkutane Absorption geeignet ist. Sie zeichnet sich dadurch aus, daß Tulobuterol sowohl in gelöster wie auch in kristalliner Form in der Haftkleberschicht des Pflasters vorhanden ist.

Schließlich wird in der EP 0 922 453 A2 eine Vorrichtung zur perkutanen Verabreichung von Tulobuterol offenbart. Diese Darreichungsform umfaßt eine Acrylathaftkleberschicht, welche mindestens 5 Gew.-% Tulobuterol als freie Wirkstoffbase in vollständig gelöstem Zustand enthält. Der aktiver Wirkstoff bleibt gelöst, so daß kein Aktivitätsverlust durch Kristallisation auftritt. Ein Hinweis auf eine mögliche Verwendung von Salzen des Tulobuterols oder gar des Hydrochlorids findet sich in EP 0 922 453 A2 nicht.

Bei den vorbeschriebenen Tulobuterol-enthaltenden transdermalen therapeutischen Systemen wird Tulobuterol vorzugsweise als freie Base eingesetzt. Der Grund dafür liegt darin, daß die freie Base wegen ihres hydrophoben Charakters gut über die Haut aufgenommen werden kann, während die Salze des Tulobuterols (z. B. Tulobuterol-hydrochlorid) stärker hydrophil sind, woraus eine schlechtere Hautgängigkeit resultiert. Aus diesem Grund wurde Tulobuterol-hydrochlorid bisher nur für die perorale Therapie verwendet, nicht aber für die transdermale Verabreichung. Lediglich in der US 5 254 348 werden in allgemeiner Weise auch die pharmazeutisch akzeptablen Salze des Tulobuterols genannt, ohne allerdings näher auf die Problematik der Hydrophilie oder der schlechteren Hautgängigkeit der Salze einzugehen.

Abgesehen von der schlechteren Hautgängigkeit bringt die Verwendung von Tulobuterol-hydrochlorid anstelle der freien Base auch einige beachtenswerte Vorteile mit sich. Erstens wird es in der Asthma-Therapie bereits viel länger und in weitaus größerem Umfang eingesetzt. Damit kann auf wesentlich umfangreicheres Material zur Toxikologie und Pharmakologie dieser Wirksubstanz zurückgegriffen werden. Zweitens ist Tulobuterol-hydrochlorid aufgrund der weltweit häufigeren Verwendung kommerziell günstiger und aus zahlreicheren Bezugsquellen erhältlich als die freie Base. Als Vorteil ist auch zu werten, daß Tulobuterol-hydrochlorid im Japanischen Arzneibuch (JP XIII) monographisch beschrieben ist. Deshalb existiert für diese Substanz - anders als für Tulobuterol-Base - bereits jetzt ein pharmazeutischer Qualitätsstandard, der für weltweite Zulassungen herangezogen werden kann.

Die Aufgabe der vorliegenden Erfindung war es deshalb, eine transdermale Darreichungsform bereitzustellen, welche die Verabreichung von Tulobuterol in Form seines Salzes Tulobuterol-hydrochlorid ermöglicht und welche die mit der Verwendung von Tulobuterol-Hydrochlorid verbundenen Vorteile aufweist. Zudem sollen mit einer solchen transdermalen Darreichungsform Hautpermeationsraten erzielt werden können, die ausreichend sind, um einen therapeutischen Einsatz zu gewährleisten.

Überraschenderweise gelingt die Lösung der Aufgabe durch ein transdermales therapeutisches System gemäß Anspruch 1. Ein erfindungsgemäßes transdermales therapeutisches System weist einen Aufbau auf, der eine weitgehend wasserdampfundurchlässige Rückschicht, mindestens eine wirkstoffhaltige Matrixschicht und eine ablösbare Schutzschicht umfaßt. Die erfindungsgemäße Arzneiform enthält Tulobuterol in Form seines Salzes Tulobuterol-hydrochlorid, wobei sich der Wirkstoff in einer Polymermatrix befindet, die auf der Basis von Polyacrylathaftklebem aufgebaut ist.

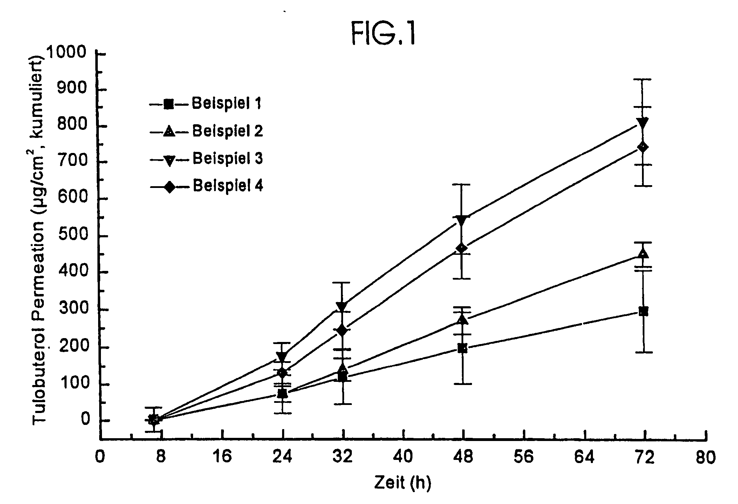

Unter Verwendung von Tulobuterol-hydrochlorid wurden mit dem erfindungsgemäßen Applikationssystem Permeationsraten von Tulobuterol durch Humanhaut in vitro von über 300 µg/cm²·d erzielt (vgl. FIG. 1). Dies ist angesichts der Tatsache, daß es sich um eine relativ hydrophile Salzform eines Wirkstoffes handelt, eine überraschend hohe Permeationsrate. Es wird angenommen, daß Tulobuterol-hydrochlorid nach seiner Freisetzung aus dem TTS in Form der freien Base Tulobuterol durch die Haut permeiert (vgl. FIG. 1).

Mit Hilfe der erfindungsgemäßen Darreichungsformen können die bei peroraler Verabfolgung gebräuchlichen Tagesdosen von 0,5 bis 6 mg Tulobuterol-hydrochlorid auch auf transdermalem Wege verabreicht werden. Vorzugsweise liegen die transdermal verabreichbaren Tagesdosen im Bereich von 2 bis 4 mg. Dabei kann sich der Applikationszeitraum über bis zu drei Tage und länger erstrecken. Somit sind die hier beschriebenen Tulobuterol-hydrochlorid enthaltenden transdermalen therapeutischen Systeme aufgrund ihrer Wirkstofffreisetzungs-Charakteristik für den therapeutischen Einsatz beispielsweise bei asthmatischen Krankheitsformen geeignet.

Der Wirkstoffgehalt der Matrix bzw. die Wirkstoffkonzentration kann in weiten Bereichen variiert werden. Bei einer bevorzugten Ausführungsform der Erfindung liegt der Massen-Gehalt von Tulobuterol-hydrochlorid in einem Bereich von 2,5 bis 20 %, vorzugsweise in einem Bereich von 5 bis 10 %, bezogen auf die gesamte Masse der wirkstoffhaltigen Matrix.

Die Grundsubstanz der Matrixschicht oder die Matrixschichten, von denen mindestens eine einen Gehalt an Tulobuterol-hydrochlorid aufweist, ist auf der Basis von Polyacrylat-Haftklebern aufgebaut oder besteht zum überwiegenden Teil aus Polyacrylat-Haftklebern. Die hierfür geeigneten Polymere oder Polymergemische sind dem Fachmann bekannt; in Frage kommen vor allem Polyacryl-, Polymethacryl-, und Polymethylmethacrylsäure und deren Derivate sowie Acrylsäureester-Copolymere. Um die mechanischen Eigenschaften der Matrix (z. B. Kohäsion, Elastizität) an spezielle Anforderungen anzupassen, können der Matrix auch noch weitere Polymere zugesetzt werden, beispielsweise Polyvinylacetat, Silikonpolymere, Polyisobutylen, Polyisopren oder styrolhaltige Blockcopolymere.

Besonders gute Ergebnisse können mit einer Ausführungsform der Erfindung erzielt werden, bei der die Polymerzusammensetzung der wirkstoffhaltigen Matrix ein Polymer mit aminofunktionellen Gruppen in den Seitenketten enthält. Der Massenanteil dieses Polymers beträgt dabei 2 bis 20 %, vorzugsweise 10 bis 16 %, bezogen auf die Masse der wirkstoffhaltigen Matrix. Vorzugsweise wird als Polymer mit aminofunktionellen Gruppen ein Butylmethacrylat-(2-Dimethylaminoethyl)-Methylmethacrylat-Copolymer verwendet, bei welchem die Monomere im molaren Verhältnis von 1:2:1 vorliegen (= Eudragit® E-100).

Im einfachsten Fall enthält das erfindungsgemäße transdermale System - neben der Rück- und Schutzschicht - nur eine einzige wirkstoffhaltige Matrixschicht. Besonders bevorzugt ist jedoch eine Ausführungsform, welche durch eine zweite Matrixschicht gekennzeichnet ist, die ebenfalls Tulobuterol-Hydrochlörid enthält. Die beiden wirkstoffhaltigen Schichten sind dabei aufeinander laminiert. Die Beladung mit Wirkstoff kann so gewählt werden, daß sie in beiden Schichten gleich hoch ist, sie kann aber auch unterschiedlich hoch gewählt werden. In der Regel werden die beiden wirkstoffhaltigen Schichten den gleichen Gehalt an Zusatzstoffen aufweisen. Jedoch kann es für bestimmte Anwendungen von Vorteil sein, wenn sich die beiden Schichten hinsichtlich ihres Gehalts an Zusatzstoffen unterscheiden. Auch können für die beiden Schichten unterschiedliche Polymerzusammensetzungen für die Matrix ausgewählt werden.

Eine weitere bevorzugte Ausführungsvariante sieht vor, daß mindestens eine der Matrixschichten im Zustand unmittelbar nach der Herstellung keinen Wirkstoff enthält. Beispielsweise kann im Falle einer zweischichtigen Matrix die hautseitige Matrixschicht wirkstofffrei hergestellt sein, während die direkt darüber befindliche zweite Matrixschicht (Reservoirschicht) Tulobuterol-hydrochlorid enthält. Nach der Herstellung und während des Anwendungszeitraums diffundiert der Wirkstoff aus dieser Schicht in die hautseitige, vorher wirkstofffreie Matrixschicht, von welcher aus die Permeation in die Haut erfolgen kann. Die Herstellung der letztgenannten Wirkstoffpflaster ist produktionslogistisch vorteilhaft, allerdings müssen etwas geringere Wirkstoff-Freisetzungsraten in Kauf genommen werden.

In bestimmten Fällen kann es von Vorteil sein, wenn die Therapie durch Tulobuterol durch die Therapie mit einem oder mehreren zusätzlichen Wirkstoffen kombiniert wird. Deshalb sind weitere Ausführungsformen vorgesehen, bei denen mindestens eine weitere Matrixschicht einen oder mehrere weitere pharmazeutische Wirkstoffe enthält.

Zur Verbesserung der Abgabe von Tulobuterol-hydrochlorid können den Matrixschichten zusätzlich hautpermeationsfördemde Zusätze zugesetzt werden. Besonders gut geeignet für diesen Zweck sind gesättigte oder ungesättigte Fettsäuren, einzeln oder in Kombination, vorzugsweise Laurinsäure, Myristinsäure oder Ölsäure. Die Matrixschicht kann einen Gehalt von 2 bis 20 %, vorzugsweise von 5 bis 10 % an Fettsäuren aufweisen, bezogen auf die Gesamtmasse der Matrixschicht. Auch Kombinationen verschiedenen Fettsäuren können vorteilhaft eingesetzt werden.
Als permeationsfördemde Zusätze eignen sich ferner auch Stoffe aus der Gruppe der niedermolekularen ein- oder mehrwertigen Alkohole, Fettalkohole, Fettalkoholether, polyoxyethylierten Fettalkohole, Fettsäureester (insbesondere Monoglyceride und Monoester mit Propylenglykol), sowie der Sorbitanfettsäureester und polyoxyethylierten Sorbitanfettsäureester.

Des weiteren können die wirkstoffhaltigen Matrixschichten Weichmacher, Klebrigmacher, kohäsionsfördemde Zusätze, Stabilisatoren, Füllstoffe und ähnliche Zusätze enthalten. Die hierfür geeigneten Stoffe sind dem Fachmann bekannt.

Gemäß einer weiteren bevorzugten Ausführungsform weist die wirkstoffhaltige Matrix der Tulobuterol-hydrochlorid enthaltenden transdermalen Wirkstoffpflaster ein Flächengewicht von mindestens 120 g/m² auf. Solche überdurchschnittlich hohen Schichtdicken der Matrix gewährleisten insbesondere eine gleichbleibend hohe Abgaberate über einen Zeitraum von mehr als 24 Stunden. Die für die im wesentlichen wasserdampfundurchlässige Rückschicht und die wiederablösbare Schutzschicht verwendbaren Materialien sind dem Fachmann bekannt. Für die Rückschicht eignen sich insbesondere Polymerfolien, vor allem Polyester, die sich durch besondere Festigkeit und Diffusionsfestigkeit auszeichnen, darüber hinaus aber nahezu beliebige andere hautverträgliche Kunststoffe, wie z. B. Polyvinylchlorid, Ethylenvinylacetat, Vinylacetat, Polyethylen, Polypropylen, Cellulosederivate und viele andere mehr. Im Einzelfall kann die Rückschicht mit einer zusätzlichen Auflage versehen werden, z. B. durch Bedampfung mit Metallen oder anderen diffusionssperrenden Zusatzstoffen wie Siliciumdioxid, Aluminiumoxid ähnlicher, dem Fachmann bekannter Stoffe. Zur Verbesserung des äußeren Erscheinungsbilds kann die Rückschicht auch auf der Außenseite hautfarben lackiert werden oder auf andere Weise behandelt werden.

Abhängig von der Festigkeit und Durchlässigkeit des gewählten Materials beträgt die Dicke der folienartigen Rückschicht gewöhnlich 8 bis 80 µm. Sie kann für spezielle Zwecke jedoch auch dicker oder dünner als diese Werte eingestellt werden.

Die wiederablösbare, vor Anwendung des Pflasters zu entfemende Schutzschicht besteht vorzugsweise aus Polyestermaterial (z. B. Polyethylenterephthalatfolie), aber auch beliebige andere hautverträgliche Kunststoffe können verwendet werden, wie z. B. Polyvinylchlorid, Ethylenvinylacetat, Vinylacetat, Polyethylen, Polypropylen oder Cellulosederivate. Im Einzelfall kann eine Bedampfung mit Metallen oder anderen diffusionssperrenden Zusatzstoffen wie Siliciumdioxid, Aluminiumoxid o. ä. vorgenommen werden. In jedem Fall ist zur Seite der klebenden Matrix hin eine Oberflächenbeschichtung mit dehäsiven Materialien erforderlich, beispielsweise mit Silikonen oder fluorhaltigen Kunststoffen, damit der Verbund leicht ablösbar bleibt.

Insbesondere nach längerer Lagerzeit kann es bei den erfindungsgemäßen Tulobuterol-HCl enthaltenden TTS zu einer leichten Gelbfärbung kommen; weiterhin wurden nach einigen Monaten geringfügige Rekristallisationen als Anzeichen einer gewissen Übersättigung mit Tulobuterol-HCl beobachtet. Zur Vermeidung dieser unerwünschten Veränderungen wurden verschiedene Zusätze aus dem Bereich der Antioxidantien und Metallionenkomplexierungsmittel getestet.

Dabei wurde gefunden, daß das Auftreten der Gelbfärbung besonders wirksam durch eine Kombination eines phenolischen Antioxidans und einer polyvalenten Säure (oder deren Salze) unterdrückt werden kann. Als Antioxidans wird vorzugsweise Butylhydroxytoluol (BHT) oder Butylhydroxyanisol (BHA) verwendet.

Als Säuren kommen vorzugsweise Citronensäure oder Ethylendiamintetraessigsäure (EDTA) oder deren Salze, wie z. B. Dinatrium-EDTA, zum Einsatz. Überraschenderweise wurde gefunden, daß durch den Zusatz der genannten Antioxidantien und Komplexbildner nicht nur das Auftreten der Verfärbung unterdrückt wird, sondern daß gleichzeitig auch eine Rekristallisation unterbleibt.

Deshalb ist gemäß der bestmöglichen Ausführungsform der Erfindung vorgesehen, daß die Tulobuterol-haltigen TTS mindestens einen Zusatzstoff aus der Gruppe der Antioxidantien, bevorzugt phenolische Verbindungen, besonders bevorzugt Butylhydroxytoluol oder Butylhydroxyanisol, sowie zusätzlich mindestens einen weiteren Zusatzstoff aus der Gruppe der Metallionen-Komplexierungsmittel, vorzugsweise Citronensäure oder Ethylendiamintetraessigsäure, besonders bevorzugt Ethylendiamintetraessigsäure-Dinatriumsalz (Na₂EDTA), enthalten.

Im folgenden soll die Erfindung anhand von Beispielen erläutert werden.
Diese Beispiele schränken die Erfindung in keiner Weise ein.

### Beispiele

Die in TAB. 1 zusammengestellten Beispiele von erfindungsgemäßen transdermalen therapeutischen Systemen weisen alle einen Aufbau aus einer Rückschicht, einer Tulobuterol-hydrochlorid enthaltenden Reservoirschicht (als erste Matrixschicht) und einer optional wirkstofffreien, hautseitigen Haftkleberschicht auf. Die hautseitige Matrix- bzw. Haftkleberschicht ist mit einer ablösbaren Schutzfolie bedeckt.
"Optional wirkstofffrei" bedeutet, daß diese hautseitige Schicht bei der Herstellung nicht mit Tulobuterol-hydrochlorid beladen wird und deshalb im Ausgangszustand wirkstofffrei ist. Nach der Herstellung und während der Anwendung findet eine Diffusion des Wirkstoffs aus der Reservoirschicht durch die wirkstofffreie hautseitige Schicht in Richtung Haut statt, gefolgt von der Permeation des Wirkstoffs durch die Haut.

Zur Herstellung der wirkstoffhaltigen Matrixschichten wurde zunächst Tulobuterol-hydrochlorid (Tulobuterol-HCl) in Ethanol gelöst. Anschließend wurden zu dieser Lösung die übrigen Komponenten, einschließlich der Matrixpolymere, in der geeigneten Menge gebräuchlicher organischer Lösungsmittel hinzugefügt.

Die organischen Lösungen wurden sodann mit einem Handfilmziehrahmen auf eine silikonisierte Polyethylenterephthalatfolie (100 µm Dicke) beschichtet und 10 min bei 80 °C in einem Abluftofen getrocknet. Die auf diese Weise erhaltene Reservoirschicht und hautseitige Schicht wurden mechanisch zusammenlaminiert, die Schutzfolie der Reservoirschicht wurde entfernt und diese Schicht mit einer Polyethylenterephthalatfolie (15 µm Dicke) als bleibende Rückschicht abgedeckt.

Die Eignung der gemäß TAB. 1 hergestellten beispielhaften Applikationssysteme für eine transdermale Therapie mit Tulobuterol wurde arn Modell der exzidierten humanen Vollhaut geprüft. Die Versuche wurden mit modifizierten Permeationszellen nach FRANZ bei 32 °C in der dem Fachmann bekannten Weise durchgeführt.
Die Messung der durch die Hautproben hindurchgetretenen Mengen von Tulobuterol erfolgte mittels HPLC. Die Ergebnisse sind in FIG. 1 dargestellt. Die angegebenen Werte stellen Mittelwert und Standardabweichung für jeweils n=3 Hautproben dar.

Die Beispiele 1 und 2 zeigen einen Aufbau, bei dem die hautseitige Schicht im Ausgangszustand wirkstofffrei ist. Nach der Herstellung erfolgt dann eine Diffusion des Wirkstoffs auch in diese Schicht. Dieser Aufbau ist produktionslogistisch vorteilhaft, die erzielten Abgaberaten durch Humanhaut in vitro sind jedoch vergleichsweise niedrig, da das System insgesamt nur eine niedrigere Gesamtbeladung mit Tulobuterol-HCl ermöglicht.

Bei den Beispielen 3 und 4 ist Tulobuterol-HCl in beiden Schichten in gleicher Konzentration enthalten. Die Gesamtbeladung ist höher als bei den Beispielen 1 und 2. Dies spiegelt sich auch in deutlich erhöhter Abgabeleistung in vitro wieder.

Beispiel 3 ist Beispiel 4 in der Abgaberate leicht überlegen. Der gegenüber Beispiel 4 leicht emiedrigte Anteil von Eudragit® E100 in der Reservoirschicht, wirkt sich offenbar gleichzeitig vorteilhaft auf die Abgabeleistung aus.

Hervorzuheben ist, daß bei allen vier Beispielen für den Wirkstoff Tulobuterol ein lineares Abgabeverhalten erreicht wird, das mindestens bis zu 72 h aufrecht erhalten wird (FIG. 1).

Beispiel 5 zeigt eine Rezeptur, welche der bestmöglichen Ausführungsform der Erfindung entspricht. Sowohl die hautseitige Schicht als auch die Reservoirschicht enthalten eine Kombination eines phenolischen Antioxidans (Butylhydroxytoluol, BHT) und Ethylendiamintetraessigsäure (Dinatriumsalz), wodurch die Gelbfärbung der TTS und die Rekristallisation des Wirkstoffs Tulobuterol während der Lagerzeit zuverlässig verhindert wird.

**TAB. 1**

| Beispiel Nr. | Flächengewicht [g/m²]** | Reservoirschicht Komponenten | % | Hautseitige Schicht Komponenten | % |
|---|---|---|---|---|---|
| 1 | 154,3 | Tulobuterol-HCl | 11,6 | Tulobuterol-HCl | ----- |
| | | Eudragit® E100 | 13,7 | Eudragit® E100 | 10,0 |
| | | Durotak® 2287* | 74,7 | Durotak® 2287* | 77,5 |
| | | Ölsäure | ----- | Ölsäure | 12,5 |
| 2 | 166 | Tulobuterol-HCl | 16,3 | Tulobuterol-HCl | ------ |
| | | Eudragit® E100 | 19,3 | Eudragit® E100 | 10,0 |
| | | Durotak® 2287* | 64,4 | Durotak® 2287* | 72,7 |
| | | Ölsäure | ----- | Ölsäure | 17,3 |
| 3 | 162,1 | Tulobuterol-HCl | 11,6 | Tulobuterol-HCl | 11,6 |
| | | Eudragit® E100 | 13,7 | Eudragit® E100 | 15,1 |
| | | Durotak® 2287* | 68,5 | Durotak® 2287* | 67,1 |
| | | Ölsäure | 6,2 | Ölsäure | 6,2 |
| 4 | 171,3 | Tulobuterol-HCl | 11,6 | Tulobuterol-HCl | 11,6 |
| | | Eudragit® E100 | 15,1 | Eudragit® E100 | 15,1 |
| | | Durotak® 2287* | 67,1 | Durotak® 2287* | 67,1 |
| | | Ölsäure | 6,2 | Ölsäure | 6,2 |
| 5 | 159,0 | Tulobuterol-HCl | 11,6 | Tulobuterol-HCl | 11,6 |
| | | Eudragit® E100 | 13,7 | Eudragit® E100 | 15,1 |
| | | Durotak® 2287* | 67,9 | Durotak® 2287* | 66,5 |
| | | Ölsäure | 6,2 | Ölsäure | 6,2 |
| | | BHT | 0,1 | BHT | 0,1 |
| | | Na₂EDTA | 0,5 | Na₂EDTA | 0,5 |

| | | | | | |
|---|---|---|---|---|---|
| * vemetzt durch Aluminiumionen (0,05 Massenprozent) | | | | | |
| ** bezogen auf die Summe von Reservoirschicht und hautseitiger Schicht. BHT=Butylhydroxytoluol; Na₂EDTA=Ethylendiamintetraessigsäure, Dinatriumsalz. Die Prozentangaben in der Tabelle bezeichnen Massenanteile (m/m), bezogen auf die Gesamtmasse der jeweiligen Matrixschicht. | | | | | |

## Patentansprüche

1. Transdermales therapeutisches System, umfassend eine weitgehend wasserdampfundurchlässige Rückschicht, mindestens eine wirkstoffhaltige Matrixschicht mit dem Wirkstoff Tulobuterol, sowie eine ablösbare Schutzschicht, **dadurch gekennzeichnet, daß** die Matrix auf der Basis von Polyacrylathaftklebern aufgebaut ist und Tulobuterol in Form seines Salzes Tulobuterol-hydrochlorid als Wirkstoff enthält.

2. Transdermales therapeutisches System nach Anspruch 1, **dadurch gekennzeichnet, daß** der Massen-Gehalt an Tulobuterol-hydrochlorid 2,5 bis 20 %, vorzugsweise 5 bis 10 % beträgt, bezogen auf die Masse der wirkstoffhaltigen Matrix.

3. Transdermales therapeutisches System nach Anspruch 1 oder 2,
**dadurch gekennzeichnet, daß** die wirkstoffhaltige Matrix ein Polymer mit aminofunktionellen Gruppen in den Seitenketten enthält und der Massenanteil dieses Polymers 2 bis 20 %, vorzugsweise 10 bis 16 % beträgt, bezogen auf die Masse der wirkstoffhaltigen Matrix.

4. Transdermales therapeutisches System nach Anspruch 3,
**dadurch gekennzeichnet, daß** als Polymer mit aminofunktionellen Gruppen ein Butylmethacrylat-(2-Dimethylaminoethyl)-Methylmethacrylat-Copolymer verwendet wird.

5. Transdermales therapeutisches System nach einem oder mehreren der vorangehenden Ansprüche, **dadurch gekennzeichnet, daß** es eine zweite Tulobuterol-hydrochlorid enthaltende Matrixschicht aufweist.

6. Transdermales therapeutisches System nach einem oder mehreren der vorangehenden Ansprüche, **dadurch gekennzeichnet, daß** es im Zustand unmittelbar nach der Herstellung mindestens eine weitere Matrixschicht auf der Basis eines Polyacrylat-Haftklebers aufweist, die keinen Wirkstoff enthält.

7. Transdermales therapeutisches System nach einem oder mehreren der vorangehenden Ansprüche, **dadurch gekennzeichnet, daß** es permeationsfördemde Zusätze enthält.

8. Transdermales therapeutisches System nach einem oder mehreren der vorangehenden Ansprüche, **dadurch gekennzeichnet, daß** es eine oder mehrere Fettsäuren in der Matrixschicht enthält, vorzugsweise Laurinsäure, Myristinsäure oder Ölsäure, wobei die Fettsäure(n) in einer Konzentration von 2 bis 20 %, vorzugsweise von 5 bis 10 % enthalten sind, bezogen auf die Gesamtmasse der Matrixschicht.

9. Transdermales therapeutisches System nach einem oder mehreren der vorangehenden Ansprüche, **dadurch gekennzeichnet, daß** es die transdermale Verabreichung der üblichen Tagesdosen von 0,5 bis maximal 6 mg, vorzugsweise von 2 bis 4 mg Tulobuterol-hydrochlorid über einen Applikationszeitraum von mindestens 3 Tagen ermöglicht.

10. Transdermales therapeutisches System nach einem oder mehreren der vorangehenden Ansprüche, **dadurch gekennzeichnet, daß** mindestens eine Matrixschicht einen oder mehrere weitere pharmazeutische Wirkstoffe enthält.

11. Transdermales therapeutisches System nach einem oder mehreren der vorangehenden Ansprüche, **dadurch gekennzeichnet, daß** das Flächengewicht der wirkstoffhaltigen Matrix mindestens 120 g/m² beträgt.

12. Transdermales therapeutisches System nach einem oder mehreren der vorangehenden Ansprüche, **gekennzeichnet durch** einen Gehalt an mindestens einem Zusatzstoff aus der Gruppe der Antioxidantien, bevorzugt phenolische Verbindungen, besonders bevorzugt Butylhydroxytoluol oder Butylhydroxyanisol.

13. Transdermales therapeutisches System nach Anspruch 12, **gekennzeichnet durch** einen Gehalt an mindestens einem weiteren Zusatzstoff aus der Gruppe der Metallionen-Komplexierungsmittel, vorzugsweise Citronensäure oder Ethylendiamintetraessigsäure, besonders bevorzugt Ethylendiamintetraessigsäure-Dinatriumsalz (Na₂EDTA).

14. Verwendung von Tulobuterol-Hydrochlorid zur Herstellung eines transdermalen therapeutischen Systems nach einem der Ansprüche 1 bis 13 zur therapeutischen Behandlung von Asthma und verwandten Krankheitsformen.

## Claims

1. Transdermal therapeutic system comprising a largely water vapour-impermeable backing layer, at least one active substance-containing matrix layer comprising the active substance tulobuterol, as well as a removable protective layer, **characterized in that** said matrix is based on polyacrylate pressure-sensitive adhesives and contains tulobuterol in the form of its salt tulobuterol hydrochloride as active substance.

2. Transdermal therapeutic system according to Claim 1, **characterized in that** the mass content of tulobuterol hydrochloride is 2.5 to 20%, preferably 5 to 10%, relative to the mass of the active substance-containing matrix.

3. Transdermal therapeutic system according to Claim 1 or 2, **characterized in that** the active substance-containing matrix contains a polymer having amino-functional groups in its side chains and that the mass content of said polymer is 2 to 20%, preferably 10 to 16%, relative to the mass of the active substance-containing matrix.

4. Transdermal therapeutic system according to Claim 3, **characterized in that** as the polymer comprising amino-functional groups a butyl methacrylate-(2-dimethylaminoethyl)-methyl methacrylate copolymer is used.

5. Transdermal therapeutic system according to one or more of the preceding claims, **characterized in that** it comprises a second tulobuterol hydrochloride-containing matrix layer.

6. Transdermal therapeutic system according to one or more of the preceding claims, **characterized in that** in the state immediately after its manufacture it comprises at least one further matrix layer based on a polyacrylate pressure-sensitive adhesive, which layer does not contain active substance.

7. Transdermal therapeutic system according to one or more of the preceding claims, **characterized in that** it contains permeation-enhancing additives.

8. Transdermal therapeutic system according to one or more of the preceding claims, **characterized in that** it contains one or more fatty acids in the matrix layer, preferably lauric acid, myristic acid or oleic acid, said fatty acid(s) being contained in a concentration of 2 to 20%, preferably of 5 to 10%, relative to the total mass of the matrix.

9. Transdermal therapeutic system according to one or more of the preceding claims, **characterized in that** it enables the transdermal administration of common daily doses from 0.5 up to maximally 6 mg, preferably from 2 to 4 mg, of tulobuterol hydrochloride over an application period of at least 3 days.

10. Transdermal therapeutic system according to one or more of the preceding claims, **characterized in that** at least one matrix layer contains one or more additional pharmaceutically active substances.

11. Transdermal therapeutic system according to one or more of the preceding claims, **characterized in that** the weight per unit area of the active substance-containing matrix is at least 120 g/m².

12. Transdermal therapeutic system according to one or more of the preceding claims, **characterized by** a content of at least one additive from the group of antioxidants, preferably phenolic compounds, more preferably butylhydroxytoluene or butylhydroxyanisole.

13. Transdermal therapeutic system according to claim 12, **characterized by** a content of at least one further additive from the group of metal ion complexing agents, preferably citric acid or ethylenediaminetetraacetic acid, more preferably ethylenediaminetetraacetic acid-disodium salt (Na₂EDTA).

14. The use of tulobuterol hydrochloride for the production of a transdermal therapeutic system according to any one of claims 1 to 13 for the therapeutic treatment of asthma and related forms of disease.

## Revendications

1. Système thérapeutique transdermique, comprenant une couche postérieure dans une large mesure imperméable à la vapeur d'eau, au moins une couche de matrice contenant une substance active, avec la substance active Tulobutérol, ainsi qu'une couche de protection amovible, **caractérisé en ce que** la matrice est élaborée à base d'auto-adhésifs de polyacrylate et contient le Tulobutérol sous forme de son sel chlorhydrate de Tulobutérol comme substance active.

2. Système thérapeutique transdermique selon la revendication 1, **caractérisé en ce que** la teneur massique en chlorhydrate de Tulobutérol est de 2,5 à 20%, de préférence 5 à 10%, par rapport à la masse de la matrice contenant la substance active.

3. Système thérapeutique transdermique selon la revendication 1 ou 2, **caractérisé en ce que** la matrice contenant la substance active contient un polymère avec des groupes à fonctionnalité amino dans les chaînes latérales et la proportion en masse de ce polymère est de 2 à 20%, de préférence de 10 à 16%, par rapport à la masse de la matrice contenant la substance active.

4. Système thérapeutique transdermique selon la revendication 3, **caractérisé en ce qu'**on utilise comme polymère avec des groupes à fonctionnalité amino un copolymère de méthacrylate de butyle-méthylméthacrylate de 2-diméthylaminoéthyle.

5. Système thérapeutique transdermique selon l'une ou plusieurs des revendications précédentes, **caractérisé en ce qu'**il contient une deuxième couche de matrice contenant du chlorhydrate de Tulobutérol.

6. Système thérapeutique transdermique selon l'une ou plusieurs des revendications précédentes, **caractérisé en ce qu'**il présente, dans l'état juste après la fabrication, au moins une autre couche de matrice à base d'un auto-adhésif de polyacrylate qui ne contient pas de substance active.

7. Système thérapeutique transdermique selon l'une ou plusieurs des revendications précédentes, **caractérisé en ce qu'**il contient des additifs favorisant la perméation.

8. Système thérapeutique transdermique selon l'une ou plusieurs des revendications précédentes, **caractérisé en ce qu'**il contient, dans la couche de matrice, un ou plusieurs acides gras, de préférence l'acide laurique, myristique ou oléique, le ou les acides gras étant contenus en une concentration de 2 à 20%, de préférence de 5 à 10%, par rapport à la masse totale de la couche de matrice.

9. Système thérapeutique transdermique selon l'une ou plusieurs des revendications précédentes, **caractérisé en ce qu'**il permet l'administration transdermique des doses journalières usuelles de 0,5 à au maximum 6 mg, de préférence de 2 à 4 mg de chlorhydrate de Tulobutérol sur un laps de temps d'administration d'au moins 3 jours.

10. Système thérapeutique transdermique selon l'une ou plusieurs des revendications précédentes, **caractérisé en ce qu'**au moins une couche de matrice contient une ou plusieurs autres substances actives pharmaceutiques.

11. Système thérapeutique transdermique selon l'une ou plusieurs des revendications précédentes, **caractérisé en ce que** le poids surfacique de la matrice contenant la substance active est d'au moins 120 g/m².

12. Système thérapeutique transdermique selon l'une ou plusieurs des revendications précédentes, **caractérisé par** une teneur en au-moins un additif du groupe des antioxydants, de préférence des composés phénoliques, de manière particulièrement préférée le butylhydroxytoluène ou le butylhydroxyanisole.

13. Système thérapeutique transdermique selon la revendication 12, **caractérisé par** une teneur en au moins un autre additif du groupe des complexants d'ions métalliques, de préférence l'acide citrique ou l'acide éthylènediaminetétraacétique, de manière particulièrement préférée le sel disodique de l'acide éthylènediaminetétraacétique (Na₂EDTA).

14. Utilisation de chlorhydrate de Tulobutérol pour la préparation d'un système thérapeutique transdermique selon l'une quelconque des revendications 1 à 13 pour le traitement thérapeutique de l'asthme et les formes de maladies apparentées.
